# EUROPEAN PATENT APPLICATION

(11) **EP 1 426 889 A1**
(43) Date of publication of application: **09.06.2004**
(21) Application number: 02758849.0
(22) Date of filing: 15.08.2002
(51) Int. Cl.: G06F 17/60

(54) **EXAMINATION REQUEST MANAGEMENT APPARATUS**

(30) Priority: 17.08.2001 JP 2001247883
(71) Applicant: ARKRAY, Inc., Kyoto-shi, Kyoto 601-8045 (JP)
(72) Inventor: OOTSUKA, Kazushi, ARKRAY, INC., Kyoto-shi, Kyoto 601-8045 (JP)
(74) Representative: Piésold, Alexander J.
(86) International application number: PCT/JP2002/008295
(87) International publication number: WO 2003/017162

(57) **Abstract**

An examination request management apparatus (1) for efficiently managing examination request in a medical institution. The apparatus (1) has an examination kind judging unit (12), an in-hospital examination request managing unit (13), and an extramural examination request managing unit (13). The examination kind judging unit (12) sorts examination items contained in an examination request message entered through an electronic card device (2) into the objective items of the in-hospital and extramural examinations with reference to an examination item master table (15). The in-hospital examination request managing unit (13) creates a worksheet for in-hospital examination for managing an in-hospital examination request in the medical institute. The extramural examination request managing unit (14)creates an extramural examination request slip for requesting an extramural examination institute for an extramural examination.

## Description

### Technical Field

The present invention relates to a system for processing requests for examinations such as a urinalysis and a blood examination in a medical institution.

### Background Art

In recent years, there have been known so-called electronic medical chart systems for use in hospitals, clinics, etc., which record the results of consultations and the like in electronic media, instead of conventionally used paper media. Such electronic medical chart systems generally have been introduced into relatively large hospitals, and bring various advantages such as: (1) the results of consultations and the like of a large number of patients can be recorded and kept efficiently; (2) a medical service fee can be calculated automatically by inputting an item(s) of the examination and/or treatment performed; (3) in response to an instruction as to administration input by a doctor, an instruction as to dispensing automatically is given to an on-site pharmacy or a prescription for off-site pharmacies automatically is issued, which reduces waiting time of patients; and (4) a reservation for a consultation or an examination made by each patient can be managed easily. Furthermore, electronic medical chart systems that can operate on personal computers have been developed recently, and such systems are utilized more and more widely in small-scale medical institutions such as medical offices of individual practitioners.

In general, university hospitals, general hospitals, and the like provided with adequate examination facilities can perform all kinds of examinations by themselves, whereas many small-scale medical institutions outsource all the examinations or some examinations that cannot be carried out with their examination facilities to outside examination institutions.

To this end, the examination institutions distribute examination request forms to the medical institutions in advance. In each medical institution, every time the necessity for an examination arises, a medical assistant (a nurse, a laboratory technician, or the like) collects a specimen and fills out the examination request form. The examination request form is collected by a delivery and collection service representative of the examination institution together with the specimen.

The above-described conventional electronic medical chart systems for use in personal computers can assist the selection of examination items by displaying a list of examination items such as GLU, PRO, BIL, URO, PH, and SG when a urinalysis is selected, for example. Unfortunately however, these conventional electronic medical chart systems do not have a function of supporting the operation of making examination requests. Therefore, every time the necessity of an examination arises, a doctor or a nurse has to undertake troublesome procedures including judging whether or not examination in his/her medical institution (on-site examination) of the selected examination item(s) is possible and filling out an examination request form to request examination by an outside examination institution (off-site examination) with regard to the examination item(s) that cannot be examined in his/her medical institution.

### Disclosure of Invention

Thus, in order to solve the above-described problem, it is an object of the present invention to provide an examination request management apparatus that enables efficient processing of examination requests in a medical institution.

In order to achieve the above object, an examination request management apparatus according to the present invention includes: an examination request input unit for inputting information regarding an examination request; an examination item data storage unit storing examination item data, the examination item data including examination item codes assigned to examination items and information for specifying an institution carrying out an examination of each examination item; an examination kind identifying unit that identifies, upon receipt of the examination request, an institution carrying out an examination of a requested examination item, based on an examination item code contained in the information regarding the examination request; an on-site examination request processing unit for creating an on-site examination procedure; and
an off-site examination request processing unit for creating information regarding an examination request to be submitted to an outside examination institution. When the examination kind identifying unit identifies the requested examination item as a subject for on-site examination, the examination kind identifying unit passes the information regarding the examination request to the on-site examination request processing unit. On the other hand, when the examination kind identifying unit identifies the requested examination item as a subject for off-site examination, the examination kind identifying unit passes the information regarding the examination request to the off-site examination request processing unit.

It is to be noted that the above-described "an institution carrying out an examination" may be a medical institution using the examination request management apparatus of the invention itself or an examination institution other than this medical institution. With the above-described configuration, when an examination request is made, an examination item contained in information regarding the examination request is sorted based on whether the item is a subject for on-site examination or off-site examination and recorded in the on-site examination procedure or the information regarding an examination request to be submitted to an outside examination institution by the examination kind identifying unit. Accordingly, it is possible to provide an examination request management apparatus that can save labor of a doctor or a medical assistant by eliminating the necessity of taking troublesome procedures such as sorting a requested examination item based on whether the item is a subject for on-site examination or off-site examination and filling out an examination request form, thereby allowing efficient processing of examination requests.

In the above-described examination request management apparatus, it is preferable that, with regard to an examination item as a subject for on-site examination, the examination item data further includes information for specifying an examination device, and the on-site examination request processing unit identifies an examination device corresponding to information regarding each examination request based on the information for specifying the examination device and records in the on-site examination procedure an order in which the examination request is processed in each examination device. With this configuration, medical assistants can carry out requested examinations efficiently by conducting the examinations in the order recorded in the on-site examination procedure.

Furthermore, in the above-described examination request management apparatus, it is preferable that with regard to a single examination item that can be examined in a plurality of institutions, the examination item data storage unit stores a plurality of pieces of examination item data, each containing an examination item code assigned uniquely to each of the plurality of institutions. With this configuration, by assigning a plurality of examination item codes to a single examination item, it becomes possible to present a plurality of examination institutions when there is an examination request for this examination item, thereby allowing the user to select an examination institution carrying out the examination, for example. As a result, the examination request can be processed in the manner corresponding to each examination institution.

Furthermore, in the above-described examination request management apparatus, it is preferable that, with regard to an examination item as a subject for off-site examination, the examination item data further contains information regarding evaluation of each outside examination institution. The information regarding the evaluation of each outside examination institution includes, for example, the examination fee of each examination item, the accuracy of examination, whether the response is good or bad, etc. With this configuration, it becomes possible to provide a doctor or the like with information for judging to which examination institution the examination should be requested.

Furthermore, in order to achieve the above object, a program according to the present invention allows a computer to execute operations of: inputting information regarding an examination request; identifying whether an examination item contained in the information regarding the examination request is a subject for on-site examination or a subject for off-site examination, based on examination item code, including examination item codes assigned to examination items and information for specifying an institution carrying out an examination of each examination item, contained in the information regarding the examination request; and recording the requested examination item in an on-site examination procedure when the requested examination item is identified as the subject for on-site examination, or recording the requested examination item in information regarding an examination request to be submitted to an outside examination institution when the requested examination item is identified as the subject for off-site examination. By making a computer read out and execute this program, an examination request management apparatus that enables efficient processing of examination requests can be provided.

### Brief Description of Drawings

FIG. 1 is a block diagram showing a schematic configuration of a system for use in medical institutions, including an examination request management apparatus and an electronic medical chart apparatus, according to Embodiment 1 of the present invention.
FIG. 2 illustrates one example of master records contained in an examination item master table of the electronic medical chart apparatus.
FIG. 3 illustrates one example of master records contained in an examination item master table of the examination request management apparatus.
FIG. 4 illustrates one example of master records contained in an examination institution master table of the examination request management apparatus.
FIG. 5 is a flowchart showing operation sequences of the examination request management apparatus.
FIG. 6 illustrates one example of a screen for assisting examination item selection, displayed on the electronic medical chart apparatus.
FIG. 7 illustrates one example of the contents of a worksheet for on-site examination.
FIG. 8 illustrates one example of master records contained in an examination item master table of an examination request management apparatus according to Embodiment 2 of the present invention.
FIG. 9 is a flowchart showing procedures for constructing the examination item master table of the examination request management apparatus according to Embodiment 2.
FIG. 10 is a block diagram showing a schematic configuration of a system for use in medical institutions, including the examination request management apparatus and an electronic medical chart apparatus, according to Embodiment 2.
FIG. 11 illustrates one example of a screen for assisting examination item selection, displayed on the electronic medical chart apparatus in Embodiment 2.

### Best Mode for Carrying Out the Invention

### (Embodiment 1)

Hereinafter, one embodiment of the present invention will be described with reference to the accompanying drawings.

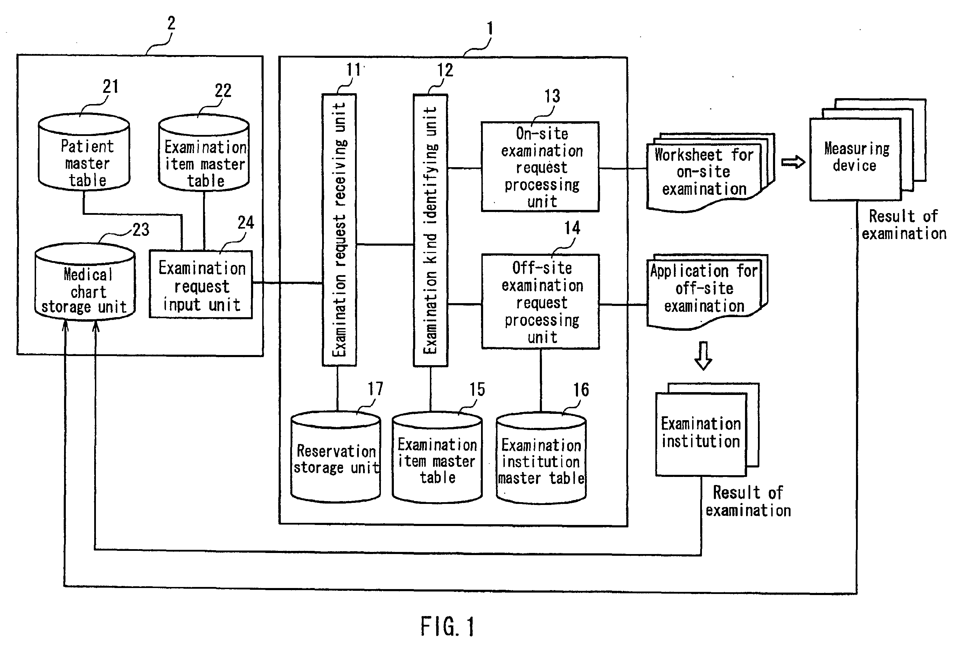

As shown in FIG. 1, an examination request management apparatus 1 according to Embodiment 1 is introduced into a medical institution employing a conventional electronic medical chart apparatus 2 and supports the electronic medical chart apparatus 2 in the tasks relating to examination request management. More specifically, the examination request management apparatus 1 creates a worksheet for on-site examination and an application for off-site examination to be submitted to an outside examination institution, in accordance with examination request information input from the electronic medical chart apparatus 2.

The examination request management apparatus 1 and the electronic medical chart apparatus 2 can be provided as independent pieces of hardware, for example, by a plurality of computers or the like. Also, it is possible to provide them in a software program that allows them to be executed as a plurality of tasks (processes) on one computer.

First, the schematic configuration of the electronic medical chart apparatus 2 will be described. Among the components of the electronic medical chart apparatus 2, only those related to the examination request management apparatus 1 according to the present embodiment are shown in FIG. 1. Thus, in addition to a patient master table 21, an examination item master table 22, a medical chart storage unit 23, and an examination request input unit 24, the electronic medical chart apparatus 2 also includes known functional units (not shown) for realizing various functions for supporting medical-action related tasks such as recording the findings on medical charts and preparing prescriptions.

The patient master table 21 stores patient information such as a name, a gender, a birth date, an address and a telephone number, the type and the number of health insurance, a patient ID and a medical chart number assigned uniquely to each patient by the medical institution, with regard to every patient.

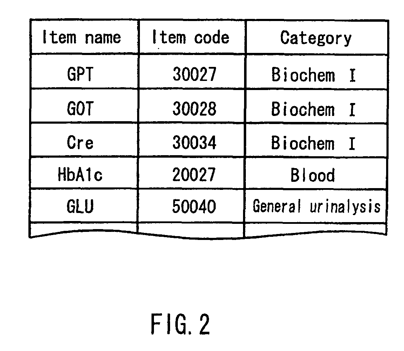

As exemplified in FIG. 2, the examination item master table 22 stores master records representing an item name, item code, etc. with regard to various examination items of a biochemical examination, a blood examination, a urinalysis, a bacteriological examination, and the like. The item code is assigned uniquely to each item at least on this electronic medical chart apparatus 2 so that the electronic medical chart apparatus 2 can perform an operation appropriate for each item. For the sake of clarity, FIG. 2 shows the master records only partially, and the arrangement of the records in this drawing is irrelevant to the order in which these master records are stored actually. Furthermore, the data structure of the master records contained in the examination item master table 22 and the contents of each record are not limited to those shown in FIG. 2 and may be designed as desired.

The examination request input unit 24 assists a doctor to select and input a necessary examination item(s) when the doctor judges, through consultation with a patient, it is necessary to carry out an examination. By this examination request input unit 24, the examination item(s) input by the doctor is passed from the electronic medical chart apparatus 2 to the examination request management apparatus 1. The medical chart storage unit 23 stores a medical chart of each patient in the form of electronic data, and the result of the examination is written on the medical chart of the patient stored in the medical chart storage unit 23.

Next, the configuration of the examination request management apparatus 1 will be described. As shown in FIG. 1, the examination request management apparatus 1 includes an examination request receiving unit 11 (examination request input unit), an examination kind judging unit 12 (examination kind identifying unit), an on-site examination request processing unit 13, an off-site examination request processing unit 14, an examination item master table 15 (examination item data storage unit), an examination institution master table 16, and a reservation storage unit 17.

The examination request receiving unit 11 receives examination request information from the electronic medical chart apparatus 2 and then performs an operation of, for example, judging whether the examination on the same day is requested or the reservation for the examination on the next day or later is made. When the examination on the same day is requested, the examination request receiving unit 11 passes the examination request information to the examination kind judging unit 12. On the other hand, when the examination on the next day or later is requested, the examination request receiving unit 11 holds the examination request information as a reservation for the examination in the reservation storage unit 17. Then, on the day of reservation, when the performance of the examination becomes certain, the examination request receiving unit 11 passes the examination request information to the examination kind judging unit 12 as a formally accepted examination request.

The examination kind judging unit 12 receives the examination request information that has been received by the examination request receiving unit 11, and judges whether each requested examination item is a subject for on-site examination or a subject for off-site examination. If the requested examination item is the subject for on-site examination, the examination kind judging unit 12 judges which examination device of the medical institution should be used, or whether the examination should be performed manually. Furthermore, when the medical institution is in alliance with a plurality of outside examination institutions, the examination kind judging unit 12 may be configured so that it judges to which examination institution the examination should be requested, as will be described later in detail.

Based on the result of the judgment made by the examination kind judging unit 12, the on-site examination request processing unit 13 and the off-site examination request processing unit 14 create a worksheet for on-site examination and an application for off-site examination, respectively. The worksheet for on-site examination and the application for off-site examination are not limited to those printed out on paper media by a printer. They may be output to recording media such as a flexible disk and the like, or may be transmitted to other computers or the like via a communication medium.

The examination item master table 15, which is different from the examination item master table 22 of the electronic medical chart apparatus 2, stores item codes of its own etc. so that an operation appropriate for each examination item is performed on the examination request management apparatus 1. The examination item master table 15 is referred to by the examination kind judging unit 12. The master records of the examination item master table 15 are created so as to suit each medical institution when introducing the examination request management apparatus 1 into the medical institution, based on the contents of the examination item master table 22 of the electronic medical chart apparatus 2 used in the medical institution and the kinds of on-site examination that can be performed in the medical institution.

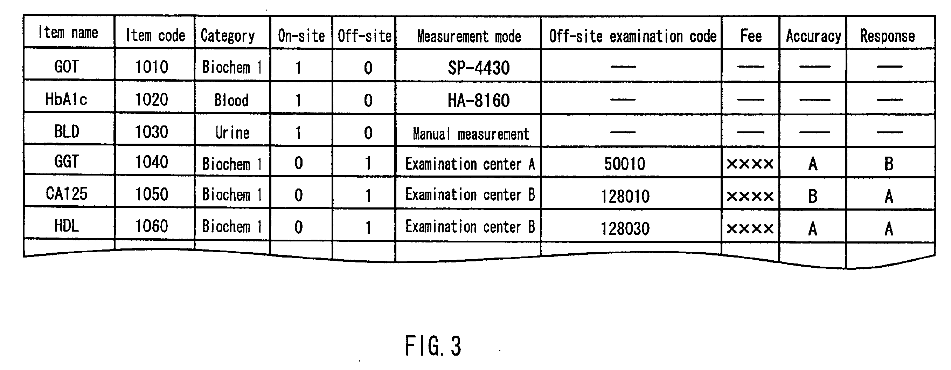

FIG. 3 illustrates one example of the master records stored in the examination item master table 15. Each record contains an item name, an item code assigned uniquely to each item on the examination request management apparatus 1, a category of an examination item, an on-site flag indicating that the examination item is a subject for on-site examination with "1", an off-site flag indicating that the examination item is a subject for off-site examination with "1", and a measurement mode indicating the kind of a measuring device to be used in on-site examination or the examination institution to which off-site examination is to be requested. Furthermore, with regard to the examination items as subjects for off-site examination, each record further contains an off-site examination code used in an examination institution to which the examination is requested, an examination fee; the accuracy of the examination by the examination institution to which the examination of the item is requested; and the time period required until the examination result is obtained (i.e., response).

Although FIG. 3 shows an example where the data stored in the examination item master table 15 are in the fixed-length record format, the structure or the contents of the data are not limited thereto, and may be designed as desired as long as it is possible to judge whether each examination item is a subject for on-site examination or off-site examination.

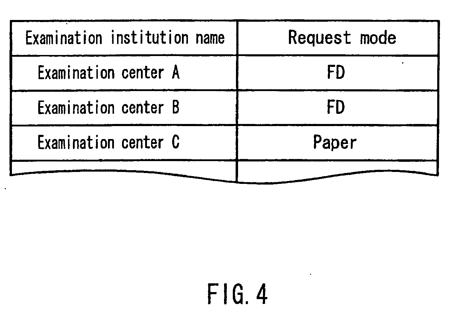

The examination institution master table 16 stores information regarding examination institutions with which the medical institution using the examination request management apparatus 1 is in alliance. The examination institution master table 16 is referred to by the off-site examination request processing unit 14. As exemplified in FIG. 4, the examination institution master table 16 stores master records containing an examination institution name and a request mode, with regard to every allied examination institution. The request mode refers to the format in which an application for off-site examination is given to each examination institution (in the example shown in FIG.4, either as a flexible disk or paper on which the application is printed out by a printer). Furthermore, the examination institution name is not limited to the actual name of an examination institution as in this example, and may be an examination institution code assigned uniquely to each examination institution at least on the examination request management apparatus 1. Also, the structure or the contents of the data stored in the examination institution master table 16 are not limited to those in the example shown in FIG. 4, and may be designed as desired. Moreover, a format of an application to be submitted to each examination institution etc. also may be stored in the examination institution master table 16.

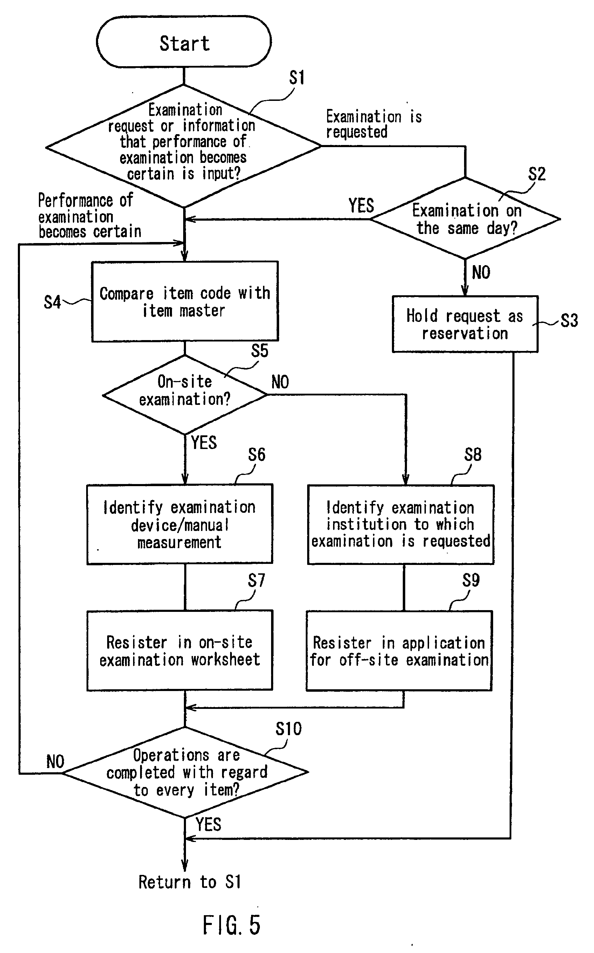

Hereinafter, the operation of the examination request management apparatus 1 will be described with reference to the flowchart shown in FIG. 5.

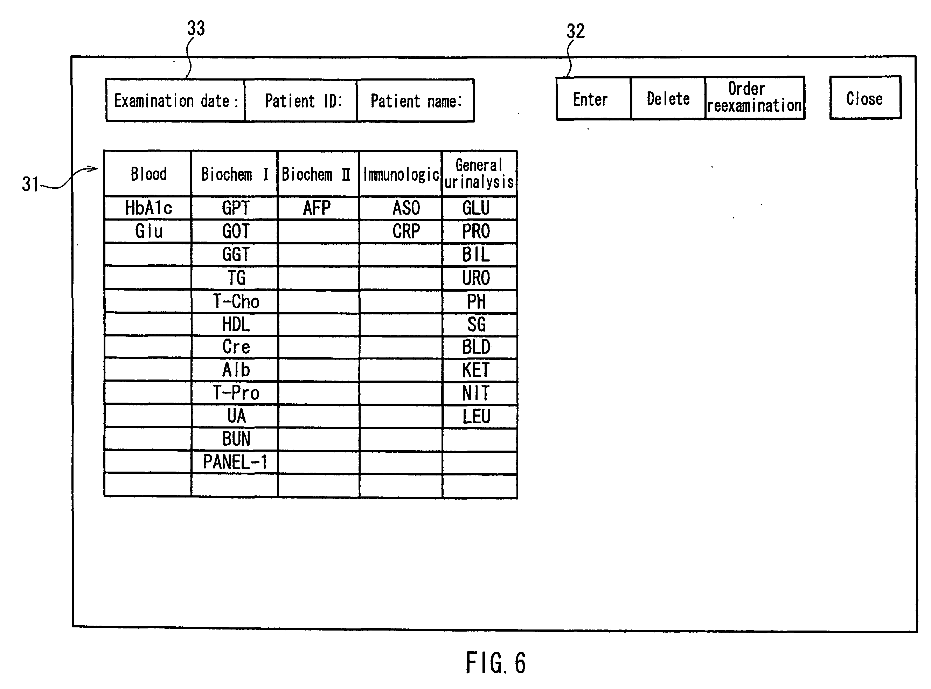

After being started up, the examination request management apparatus 1 waits for examination request information or information that a reservation for an examination is fixed is input from the electronic medical chart apparatus 2 (Step S1). In the electronic medical chart apparatus 2, a screen for assisting examination item selection as shown in FIG. 6, for example, is displayed on a display by the examination request input unit 24. From this screen, a doctor can select and input the necessary examination item(s). For example, in the case of the screen shown in FIG. 6, if the doctor clicks the cell(s) corresponding to the necessary examination item(s) in an examination item list 31 and then clicks an enter button 32, the examination request input unit 24 creates an examination request message with regard to the examination item(s) in the clicked cell(s) and transmits this message to the examination request management apparatus 1. The examination request message contains information such as a request number, an examination date, a patient ID, a patient name, one or more examination items selected by the doctor, and an item code of each examination item.

As described above, in the electronic medical chart apparatus 2, the reservation for an examination on the next day or later can made by inputting a desired date to an examination date box 33 on the screen shown in FIG. 6, for example. In this case, by performing an operation of fixing the reservation using the electronic medical chart apparatus 2 on the day of reservation, information confirming the performance of the examination is input from the examination request input unit 24 to the examination request management apparatus 1.

When the examination request information is input from the electronic medical chart apparatus 2 in Step S1, the examination request receiving unit 11 judges whether the designated examination date is the same day or not (Step S2). If the designated examination date is the same day, the examination request receiving unit 11 passes the examination request information to the examination kind identifying unit 12. On the other hand, if the designated examination date is the next day or later, the examination request receiving unit 11 holds the examination request information in the reservation storage unit 17 (Step S3).

In Step S4, the examination kind identifying unit 12 searches the examination item master table 15 for the item code of each examination item contained in the examination request message received by the examination request receiving unit 11. Then, when the examination kind identifying unit 12 finds the master record containing the same examination item code as that of the examination item, it judges whether the examination item is a subject for on-site examination or off-site examination, with reference to the on-site flag and the off-site flag of the master record.

When the examination item is judged to be the subject for on-site examination (YES in Step S5), the examination kind identifying unit 12 further judges the kind of an examination device to be used (including a manual measurement), with reference to the "measurement mode" field of the corresponding master record(s) (Step S6). Then, the examination kind identifying unit 12 passes the result of the judgment to the on-site examination request processing unit 13.

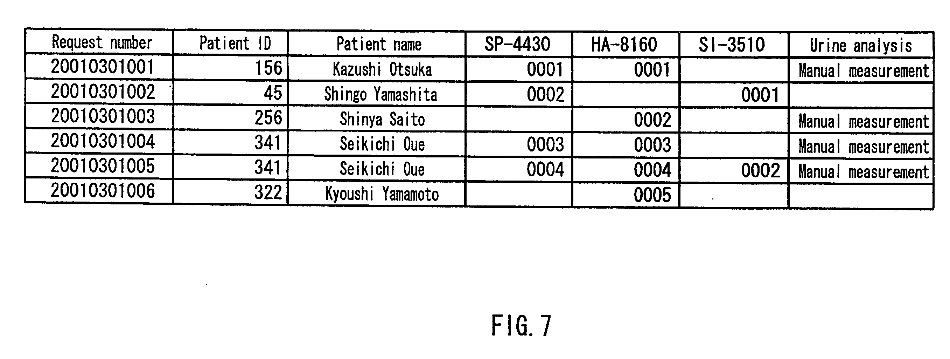

Based on the information passed by the examination kind identifying unit 12, the on-site examination request processing unit 13 creates a worksheet for on-site examination as shown in FIG. 7 (Step S7). The worksheet includes, with regard to each one piece of examination request information received by the examination request receiving unit 11, fields showing a request number, a patient ID, and a patient name, respectively, and also fields showing the order in which the requested examination should be performed in each examination device of the medical institution or by a manual measurement. Accordingly, when the on-site examination request processing unit 13 receives the kind of an examination device to be used for the examination of the item(s) contained in each piece of examination request information from the examination kind identifying unit 12, it determines the order in which the examination of each item is performed in each examination device. Specifically, in the example shown in FIG. 7, it is determined that, in the examination device named SP-4430, the specimens with the patients IDs 156, 45, 341, and 341 should be examined in this order.

On the other hand, in Step S5, when the examination item is judged to be the subject for off-site examination, the examination kind identifying unit 12 further judges to which examination institution the examination is to be requested, with reference to the "measurement mode" field of the corresponding master record (Step S8), and then passes the result of the judgment to the off-site examination request processing unit 14. The off-site examination request processing unit 14 creates an application for off-site examination to be submitted to the examination institution, in accordance with the request mode appropriate for the examination institution, with reference to the examination institution master table 16.

The operations from Step S4 through Step S9 described above are repeated until all the examination items contained in the examination request information received from the electronic medical chart apparatus 2 are processed (Step S10).

As described above, the examination request management apparatus 1 creates a worksheet for on-site examination and applications for off-site examination, based on the examination request information input from the electronic medical chart apparatus 2. Then, medical assistants of the medical institution carry out the examinations using the examination devices of the medical institution, in accordance with the worksheet created by the examination request management apparatus 1. On the other hand, the applications for off-site examination created by the examination request management apparatus 1 are given to the delivery and collection service representatives of the respective examination institutions together with the specimens. These specimens are examined in the respective examination institutions. Then, the results of the on-site and off-site examinations are registered in the medical chart storage unit 23 of the electronic medical chart apparatus 2 via communication lines or data recording media or by being input manually, for example, so that doctors can utilize the results for diagnoses.

As described above, the examination request management apparatus 1 according to the present embodiment receives the examination items selected in the electronic medical chart apparatus 2, sorts them into subjects for on-site examination and subjects for off-site examination, and creates a worksheet for on-site examination with regard to the items as the subjects for on-site examination and applications for off-site examination with regard to the items as the subjects for off-site examination.

Therefore, in the medical institution using this examination request management apparatus 1, all that is required is selecting necessary examination items on the electronic medical chart apparatus 2. Since doctors or medical assistants do not have to judge whether or not on-site examination of the examination items is possible or to which examination institution the examination should be requested as required conventionally, efficient consultations become possible.

### (Embodiment 2)

The difference between Embodiment 2 and Embodiment 1 lies in the following point. That is, in the present embodiment, on the screen from which a doctor selects examination items in an electronic medical chart apparatus 2, examination items as subjects for on-site examination and examination items as subjects for off-site examination are displayed separately, or examination items that can be examined in each institution are displayed on an institution-by-institution basis, based on the contents of an examination item master table 15 of an examination request management apparatus 1.

Furthermore, in the present embodiment, in the case where both on-site examination and off-site examination are possible with regard to a single examination item, a doctor or a medical assistant can decide whether the examination item is to be examined by the medical institution using the apparatus itself or by an outside examination institution.

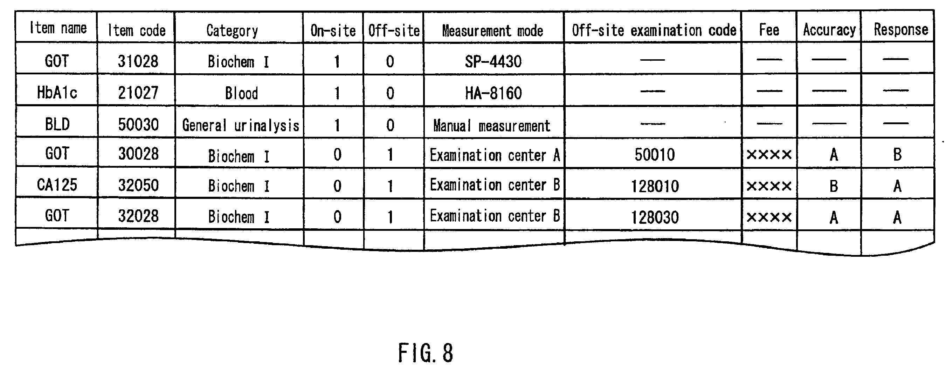

Thus, in the examination request management apparatus 1 of the present embodiment, with regard to each item that can be examined in a plurality of institutions including the medical institution itself and outside examination institutions, the examination item master table 15 stores a plurality of master records containing item codes assigned uniquely to each of the plurality of institutions. Specifically, in the example shown in FIG. 8, with regard to an item GOT, the following three master records are registered in the examination item master table 15: the master record containing an item code "31028" indicating that the item is a subject for on-site examination; the master record containing an item code "30028" indicating that the item is a subject for off-site examination performed in the examination center A, and the master record containing an item code "32028" indicating that the item is a subject for off-site examination performed in the examination center B.

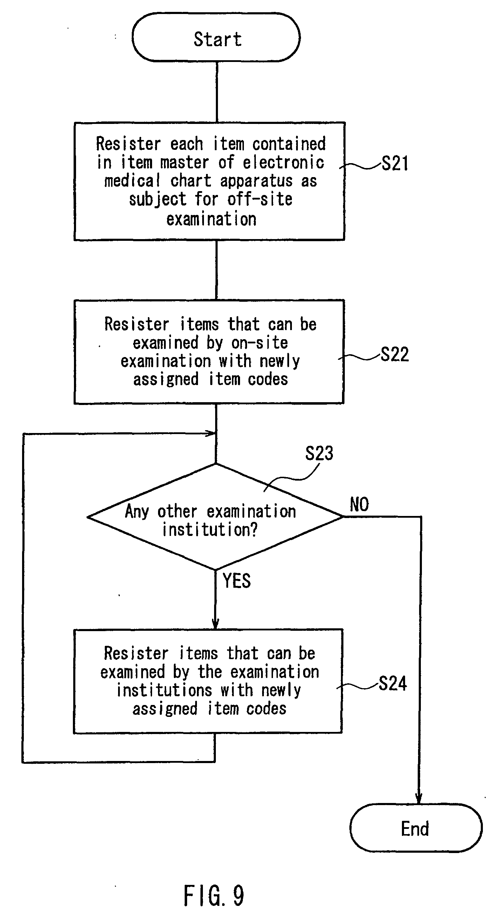

In the following, procedures for constructing the examination item master table 15 when introducing the examination request management apparatus 1 into the medical institution will be described with reference to FIG. 9. As in Embodiment 1, the examination item master table 15 according to the present embodiment also is constructed so as to suit each medical institution when introducing the examination request management apparatus 1 into the medical institution.

As can be seen from FIG. 8, each master record of the examination item master table 15 contains an item name, an item code, and a category Thus, first of all, all the master records contained in an examination item master table 22 of the electronic medical chart apparatus 2 are extracted, and the item name, item code, and category contained in each of the extracted master records are registered as the item name, the item code, and the category contained in the master records of the examination item master table 15, respectively. At this time, "0" is registered as an on-site flag and "1" is registered as an off-site flag of each master record. Furthermore, in the "measurement mode" field of each master record, a main allied examination institution (e.g., the "examination center A") is registered as an initial value. By these operations, all the examination items registered in the examination item master table 22 of the electronic medical chart apparatus 2 are registered in the examination item master table 15 temporarily as subjects for off-site examination (Step S21).

Next, the examination items that can be examined in the medical institution are clarified, and with regard to these items, new master records as subjects for on-site examination are created and registered in the examination item master table 15 (Step S22). To the master records created by the above operation, item codes different from those used in the examination item master table 22 of the electronic medical chart apparatus 2 are assigned, and the on-site flag is set to "1" and the off-site flag is set to "0". Furthermore, in the "measurement mode" field of each of these new master records, the name of the examination device of the medical institution to be used for the examination of the corresponding item is registered

Then, if the medical institution is in alliance with a plurality of examination institutions, new master records with regard to examination items that can be examined in each of the examination institutions are created and registered in the examination item master table 15 (Steps S23 and S24). To the master records created by the above operation, item codes different from those in the examination item master table 22 of the electronic medical chart apparatus 2 and from those assigned to the items as subjects for on-site examination in Step S22 are assigned, and the on-site flag is set to "0" and the off-site flag is set to "1". Furthermore, the examination institution name is registered in the "measurement mode" field of each of these new master records.

In the above-described manner, the examination item master table 15 of the examination request management apparatus 1 is customized so as to suit each medical institution. In the examination item master table 15 thus constructed, there are a plurality of master records containing item codes distinct from each other with regard to the item that can be examined in a plurality of institutions, e.g., the item GOT in FIG. 8.

In the above, an example is described where all the item codes contained in the examination item master table 22 of the electronic medical chart apparatus 2 are registered in the examination item master table 15 as subjects for off-site examination. However, for example, when there are more examination items as subjects for on-site examination than those as subjects for off-site examination, it is more effective to register all the item codes contained in the electronic medical chart apparatus 2 as subjects for on-site examination, contrary to the above-described example.

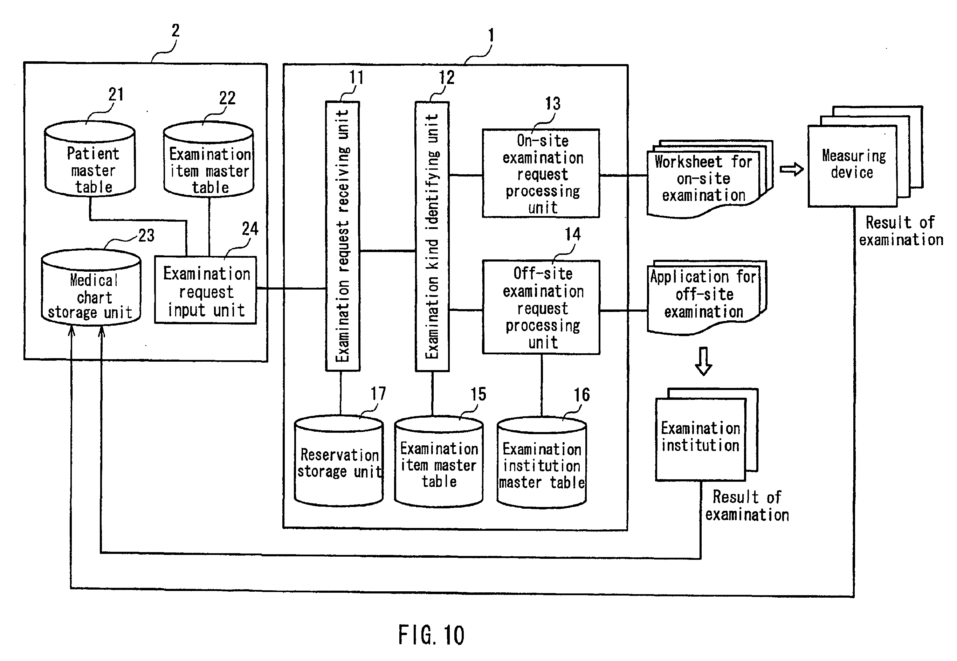

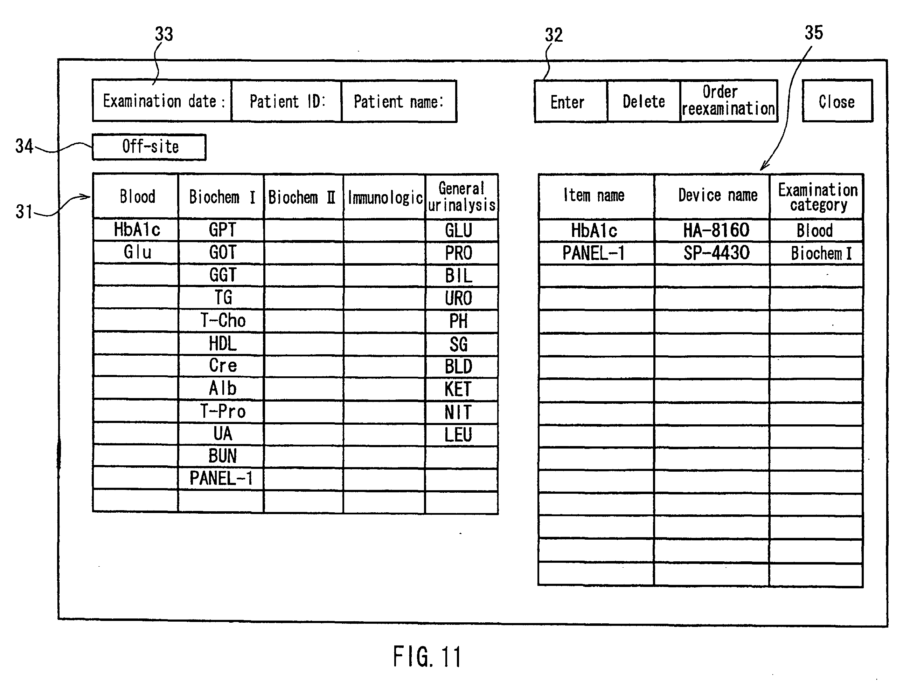

Furthermore, in the present embodiment, after the examination request management apparatus 1 is introduced into the medical institution, the examination request input unit 24 of the electronic medical chart apparatus 2 refers to the examination item master table 15 of the examination request management apparatus 1, as shown in FIG. 10. This allows the electronic medical chart apparatus 2 to display a screen as shown in FIG. 11 on a display, when a doctor selects examination items. That is, on the screen shown in FIG. 11, only the examination items as subjects for on-site examination are displayed in an examination item list 31. If the doctor clicks necessary examination items, the examination request input unit 24 searches the examination item master table 15 for the master records of the selected examination items, and the name of the examination devices to be used and the categories of the examination items are displayed in a selected item list 35. Then, if the doctor clicks an enter button 32, the items displayed on the selected item list 35 are accepted as the items for which examination requests are to be made, and transmitted to the examination request management apparatus 1 from the examination request input unit 24.

In FIG. 11, if a display-switching bottom 34 currently indicating "Off-site" is clicked, the currently displayed screen is switched into a screen displaying only examination items as subjects for off-site examination. Instead of displaying the subjects for on-site examination and the subjects for off-site examination on separate screens as in the above, whether or not on-site examination and off-site examination are possible may be displayed on an item-by-item basis.

If desired, as shown in FIG. 8, the examination fee, the accuracy of examination, and the response of the examination institutions may be registered in the master record of each examination item as a subject for off-site examination, so that, when a certain examination item can be examined in a plurality of examination institutions, a list of the examination fee, the accuracy of examination, the response, etc. of these examination institution is displayed. This allows a doctor or the like to judge to which examination institution the examination of the item should be requested easily and appropriately.

The above-described respective embodiments are not intended to limit the present invention in any way, and various changes and modifications may be made without departing from the scope of the invention. For example, while the above description has described the system configuration in which examination request information is input from the electronic medical chart apparatus 2 to the examination request management apparatus 1, the system may be configured so that an examination request is input directly to the examination request management apparatus 1 without the intermediary of the electronic medical chart apparatus 2.

### Industrial Applicability

As specifically described above, according to the present invention, it is possible to provide an examination request management apparatus that enables efficient processing of examination requests.

## Claims

1. An examination request management apparatus comprising:
an examination request input unit for inputting information regarding an examination request;
an examination item data storage unit storing examination item data, the examination item data including examination item codes assigned to examination items and information for specifying an institution carrying out an examination of each examination item;
an examination kind identifying unit that identifies, upon receipt of the examination request, an institution carrying out an examination of a requested examination item, based on an examination item code contained in the information regarding the examination request;
an on-site examination request processing unit for creating an on-site examination procedure; and
an off-site examination request processing unit for creating information regarding an examination request to be submitted to an outside examination institution,
wherein when the examination kind identifying unit identifies the requested examination item as a subject for on-site examination, the examination kind identifying unit passes the information regarding the examination request to the on-site examination request processing unit, whereas when the examination kind identifying unit identifies the requested examination item as a subject for off-site examination, the examination kind identifying unit passes the information regarding the examination request to the off-site examination request processing unit.

2. The examination request management apparatus according to claim 1, wherein with regard to an examination item as a subject for on-site examination, the examination item data further includes information for specifying an examination device, and
the on-site examination request processing unit identifies an examination device corresponding to information regarding each examination request based on the information for specifying the examination device and records in the on-site examination procedure an order in which the examination request is processed in each examination device.

3. The examination request management apparatus according to claim 1 or 2, wherein with regard to a single examination item that can be examined in a plurality of institutions, the examination item data storage unit stores a plurality of pieces of examination item data, each containing an examination item code assigned uniquely to each of the plurality of institutions.

4. The examination request management apparatus according to any one of claims 1 to 3, wherein with regard to an examination item as a subject for off-site examination, the examination item data further contains information regarding evaluation of each outside examination institution.

5. A program for allowing a computer to execute operations of:
inputting information regarding an examination request;
identifying whether an examination item contained in the information regarding the examination request is a subject for on-site examination or a subject for off-site examination, based on examination item code, including examination item codes assigned to examination items and information for specifying an institution carrying out an examination of each examination item, contained in the information regarding the examination request; and
recording the requested examination item in an on-site examination procedure when the requested examination item is identified as the subject for on-site examination, or recording the requested examination item in information regarding an examination request to be submitted to an outside examination institution when the requested examination item is identified as the subject for off-site examination.

6. The program according to claim 5, wherein with regard to an examination item as the subject for on-site examination, the examination item data further includes information for specifying an examination device, and
an examination device corresponding to information regarding each examination request is identified based on the information for specifying the examination device and an order in which the examination request is processed in each examination device is recorded in the on-site examination procedure.

7. The program according to claim 5 or 6, wherein with regard to a single examination item that can be examined in a plurality of institutions, the examination item data storage unit stores a plurality of pieces of examination item data, each containing an examination item code assigned uniquely to each of the plurality of institutions.

8. The program according to claim 7, wherein with regard to an examination item as the subject for off-site examination, the examination item data further contains information regarding evaluation of each outside examination institution.
